Europäisches Patentamt

European Patent Office

Office européen des brevets

(19)

(11) Publication number: **0 143 648**
**A2**

(12) # EUROPEAN PATENT APPLICATION

(21) Application number: **84308240.5**

(22) Date of filing: **28.11.84**

(51) Int. Cl.⁴: **C 12 N 5/00**

(30) Priority: **29.11.83 GB 8331803**

(43) Date of publication of application: **05.06.85 Bulletin 85/23**

(84) Designated Contracting States: **DE FR GB SE**

(71) Applicant: **NATIONAL RESEARCH DEVELOPMENT CORPORATION, 101 Newington Causeway, London SE1 6BU (GB)**

(72) Inventor: **Macleod, Alexander James, 167, Mayfield Road, Edinburgh EH9 3AY (GB)**

(74) Representative: **Percy, Richard Keith, Patent Department National Research Development Corporation 101 Newington Causeway, London SE1 6BU (GB)**

(54) **Process of preparing a component of a cell growth medium.**

(57) The tissue culture of mammalian cells requires a serum component, such as foetal calf serum, which is expensive. It has now been found possible to replace part or all of the serum component by an extract from human blood plasma, particularly by a cold ethanol precipitation fraction obtainable between Cohn fraction II + III combined and Cohn fraction V or similar, but only if it is processed correctly. The invention provides such a process, which comprises chilling, homogenising, raising the pH to dissolve proteins and bringing the preparation to a physiologically tolerable pH and sterilising it.

The invention is particularly application to culture of immortal cells, e.g. hybridomas, and includes the cultivated cells and cell products, in particular monoclonal antibodies, thus obtained.

126233

## PROCESS OF PREPARING A COMPONENT
## OF A CELL GROWTH MEDIUM

This invention relates to a process of preparing a component of a cell growth medium, suitable for use in the tissue culture of mammalian cells. It is particularly applicable to the culture of immortal mammalian cells as a replacement for part or all of the fetal calf serum currently used.

The nutritional requirements of mammalian cells are more stringent than those of most micro-organisms and have not yet been fully defined. Rather than being a free-living organism, a mammalian cell is adapted to a specialised life as part of an organised tissue, dependent on the specialised functions of many other cells and on a circulatory system that ensures a precisely adjusted and stable environment for each cell. Such a cell resists being separated from its tissue and grown in an artificial medium. Most animal cells will not grow at all in suspension; they grow only when they can attach themselves to a surface. However, techniques have been developed for growing mammalian cells on a small scale in the laboratory.

A mammalian cell culture begins with a mammalian tissue. The tissue is dissociated either mechanically or enzymatically, or by a combination of the two methods, to yield a mixture of single cells and small clumps of cells. The mixture is inoculated into an appropriate liquid growth medium that ordinarily includes salts, glucose, certain amino acids, vitamins and blood serum (usually accounting for between 5 and 20% of the medium).

The serum is included as a means of providing components that have not yet been identified but have been shown to be needed if the cells are to live and grow in an artificial medium. Foetal calf serum, which is considered the best available serum for this purpose, is expensive, and its cost largely determines the economic feasibility of a particular culture. Also, there is the problem that the products obtained from cells grown in FCS might be contaminated by bovine proteins, which would be undesirable for human clinical application of the cell products.

- 2 -

Although most mammalian cells need to be anchored to a solid surface, cells that originate in blood or lymphatic tissue, along with most tumour cells and other immortal cells, can be adapted to growth in suspension.

The term "immortal cells" as used in this specification refers to cells which are inherently immortal, for example the tumour-producing myeloma cells, and those which are immortalised, e.g. mammalian cells from various parts of the body which are fused with myeloma cells to give immortal hybridoma cells. Hybridoma cells are, of course, important for their use in producing monoclonal antibodies. Other immortalised cells are those which are transformed by certain viruses, for example, Epstein-Barr virus or are transformed by chemical mutagenenis. Namalwa cells, which have been used in the production of interferons, are an example of an inherently immortal cell line arising spontaneously from a tumour found in a girl.

Hitherto immortal cells have had to be grown in a medium comprising a defined component such as RPMI 1640 and an undefined component such as FCS. FCS is, however, expensive.

It would be advantageous to replace serum by some product obtainable from the processing of mammalian blood, particularly human blood. Human blood is processed in large quantities from supplies generated by the blood transfusion service. It is used to prepare fractions of blood plasma, for example, to produce albumins, immunoglobulins and supplies of proteins essential for remedying deficiencies in the blood-clotting chain.

Human blood plasma is conveniently fractionated by the cold ethanol precipitation method. For this method the plasma is treated with ethanol at below $5^{o}C$ and by increasing the concentration of ethanol and reducing the pH, a succession of fractions are precipitated. The supernatant liquor after each precipitation is subjected to the next. The whole technology is normally described by the umbrella term "Cohn precipitation". It is based on the methods described by E.J. Cohn et al., J. Amer. Chem. Soc., 68, 459-475 (1946), but the original methods therein described have been modified over the years. As an example, see J.G. Watt, Clinics in Haematology, $\underline{5}$, 95-112 (1976) and P. Kistler and H. Friedli in "Methods of Plasma Protein Fractionation", edited by J.M. Curling, Academic Press (1980), pages 3-15. Broadly, the first fraction precipitated contains fibrinogen and fibronectin, the second gamma-globulins, the third beta-globulins, the fourth alpha-globulins and the fifth albumins. The fifth fraction is a large volume product collected at a 40% ethanol concentration and pH 5.2 and represents a commercially valuable product. Each fraction is heavily contaminated by components of other fractions and other materials. Fraction IV can be recovered under various different conditions to give different products. For instance fraction IV can be collected as two cuts, known as IV(1) and IV(4), the latter containing more transferrin and albumins.

Many attempts have been made to use components of human blood plasma as a substitute for serum in the culture of mammalian cells. The following account is a selection of the prior art, believed to be representative of various lines of research, in approximate chronological order.

K. Lambert and S.J. Pirt, Develop. biol. Standard, $\underline{37}$, 63-66 (1977) (Joint WHO/IABS Symposium on the standardisation of cell substrates for the production of virus vaccines, Geneva, December 1966), reviewed the problems of trying to replace serum as a component of the growth medium for growing human diploid cells. These workers tested the growth factor activity of protein fractions of blood from the Cohn precipitation method. They reported that although growth factor can be recovered, particularly in Cohn fraction IV, the method is poorly reproducible and the fractions could not replace whole serum. They speculated that non-suppressible insulin-like activator (NSILA) or somatomedins contained in blood plasma might have a role as replacements for serum in growing human diploid cells.

J.L. Melnick and C. Wallis, Develop. biol. Standard, $\underline{37}$, 77-82, (1977) pursued the theory that an important function of serum in growth media is its ability to inhibit the proteolytic enzyme

- 4 -

trypsin. They tested this theory by trying various media as inhibitors of trypsin. These included albumin, human blood plasma Cohn fractions IV, IV(1) and IV(4), fetuin, beta-globulin gamma-globulin and calf sera. Cells washed thoroughly and cultivated without serum grew to monolayers efficiently only in the presence of serum fraction IV(4) or fetuin.

N.N. Iscove and F. Melchers, Journal of Experimental Medicine, 147, 923-933 (1978) suggested that a mixture of albumin, transferrin and lipids could replace serum entirely for support of lipopolysaccharide (LPS) - stimulated mouse B lymphocytes. However, these results were obtained by the conjoint use of a new medium, a modification of Dulbecco's modified Eagle's medium (DMEM), richer in amino acids and vitamins than the conventional medium RPMI 1640. The cells also required LPS for induction of growth from the resting phase and for their maturation to the stage at which immunoglobulin is secreted.

K. Lambert and S.J. Pirt, J. Cell Science, 35, 381-392 (1979), experimented with an ultrafiltrate fraction of calf serum. This material, molecular weight of 10,000 Daltons or less, supported growth of human diploid cells. It was concluded that a mixture of growth factors such as non-suppressible insulin-like activator (NSILA), somatomedins, epidermal growth factor (FGF) might be responsible.

A thorough study of the use of human blood plasma fractions as growth-stimulating media was made by H. Spieker-Polet et al., Cellular Immunology, 44, 144-156 (1979). These workers found that mouse and rat lymphocytes stimulated by the mitogen concanavalin A showed little growth in a protein-free medium, ample growth when albumin was included in the medium, some growth when human blood plasma Cohn fraction VI was included instead of albumin, but little or no growth from Cohn fraction II, III or IV.

The work of Melnick and Wallis, supra, was pursued by A.J. MacLeod and O. Drummond, Develop. biol. Standard, 46, 17-20 (1980). These authors analysed various sera and some protein solutions prepared from the by-products of human blood plasma

- 5 -

fractionation. A fraction IV(4) solution and a solution prepared from plasma depleted in coagulation factor, but otherwise whole, were reported to support cell growth at high cell densities. The kind of cells is not stated but were in fact skin fibroblasts.

An article by A.J. MacLeod, Nature, 285, 136-137 (1980) discussed ways of producing human proteins including the use of recombinant DNA technology and transformation of mammalian cells including preparation of hybridoma cell lines. The author suggests that by-products of the fractionation of human plasma could provide material for supporting the growth of human cells cultured in vitro. No more specific suggestion is given, but the author comments that that by-products consist largely of alpha- and beta-globulins and low molecular weight proteins and these have already been shown to contain many of the specific components required for the culture.

R. Weinstein et al., Journal of Cellular Physiology, 110, 23-28 (1982) describe a serum-free medium for cultivating normal human foreskin fibroblasts (NHFF) in vitro. These workers experimented with a medium containing acid- and heat-extracted Cohn fraction IV. The acid- and heat-extraction steps are for the purposes of producing somatomedins. (The paper refers to this material simply as "CF IV", but it is clear that this heat- and acid-extracted material, rather than the complete fraction, is intended). The authors found that NHFF growth did not decrease when the "CF IV" somatomedins were omitted. In the end, they recommended a medium which they designated "Medium F", lacking "CF IV" and consisting of insulin, transferrin, thrombin, hydrocortisone, ovalbumin, ascorbic acid, trace elements and DMEM.

T. Kawamoto et al., Analytical Biochemistry, 130, 445-453 (1983), describe a serum-free medium for cultivation of mouse myeloma cells which they designate "KSLM" and which consists of a 2:1:1 mixture (by volume) of RPMI 1640, DMEM, another defined medium known as Ham's F12, with L-glutamine, sodium pyruvate, glucose, penicillin, ampicillin and streptomycin, supplemented with crystalline bovine insulin, transferrin (free of $Fe^{3+}$ ions), 2-aminoethanol, 2-mercaptoethanol, sodium selenite, human low-density lipoprotein and oleic acid in a complex with crystalline fatty acid-free BSA. This work follows in the line of research of Iscove and Melchers, supra.

Recently C.A. Conover et al., Journal of Cellular Physiology, 116, 191-197 (1983) studied growth and replication of human fibroblasts in the presence of human hypopituitary serum (HHS). Experiments were conducted with a medium containing a sub-fraction of Cohn fraction IV(1), this sub-fraction containing insulin-like growth factor-1, otherwise known as somatomedin C (SM-C). Cells were grown in DMEM containing 20% fetal calf serum (FCS), the FCS being replaced by various amounts of SM-C. The SM-C was not by itself (in the absence of HHS) a substitute for the FCS. In the presence of 1% HHS, the SM-C did sustain cell growth, although not as well as FCS.

It will be apparent that there is a great deal of contradiction and uncertainty in the prior art in relation to the role of blood plasma constituents. For example, some researchers suggest that Cohn fraction IV(4) promotes cell growth, e.g. MacLeod and Drummond (1980) and Melnick and Wallis (1977), other authors have concentrated attention on other sub-components of fraction IV such as somatomedins, e.g. Lambert and Pirt (1977 and 1979), and Conover (1983), while Spieker-Polet (1979) et al. reject factor IV as yielding a useful growth factor and Weinstein (1982) et al. reject fraction IV a component of their optimum serum-free product.

It has now surprisingly been found that when cold ethanol precipitation fractions equivalent to Cohn fractions II + III combined, III, IV(1) or IV(4) are processed in a certain way, the product is suitable directly, without the necessary addition of other sub-components of plasma or of growth factors present in serum, as a serum-free component of a cell growth medium for mammalian cell growth. However, neither fraction II nor fraction V gives a useful product.

The processing of the fractions involves as essential steps:-

1. keeping the temperature of the chilled fraction from rising excessively;

2. homogenising the fraction;

3.  raising its pH so that proteins are re-dissolved; and

4.  sterilising the product.

According to an important feature of the invention there is provided a process for the preparation of a component of a cell growth medium, useful as a supplement to a defined medium, for the growth of mammalian cells in vitro, which comprises:-

    (a)   extracting from mammalian, especially human, blood plasma by cold ethanol precipitation a fraction of defibrinated plasma containing not more than 85%, preferably not more than 75% and even more preferably not more than 65% of albumin, and containing not more than 65% of gamma-globulins, all these percentages being reckoned by total weight of protein in the fraction;

    (b)   chilling the extract to hold it a temperature of below 10°C until homogenisation by step (c) below has been carried out;

    (c)   homogenising the chilled material;

    (d)   raising the pH of the homogenate in order to re-dissolve substantially all the precipitated protein; and

    (e)   bringing the preparation to a physiologically tolerable pH for mammalian cell growth and sterilising it (in either order).

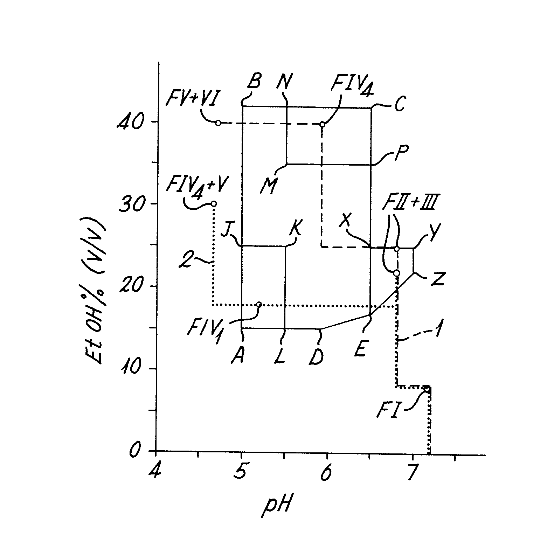

The extraction procedure using cold ethanol precipitation involves reducing pH and increasing the ethanol concentration and collecting the precipitates obtained after each such step. Various combinations of pH and ethanol concentration can be used to precipitate similar Cohn fractions and each Cohn fraction of a given numerical designation represents the precipitate obtained with a small range of pH at a given ethanol concentration and a small range of ethanol concentrations at a given pH. Therefore, to explain preferred embodiments of the invention more precisely, reference is made to the accompanying drawing which is a matrix or two-dimensional plot of pH on the abscissa and ethanol concentration on the ordinate, whereby the Cohn fractions believed to be of particular value in the invention can be conveniently defined in terms of pH and ethanol concentration.

Referring now to the accompanying drawing, the broken line 1 and the dotted line 2 represent two different regimes of Cohn precipitation. Both begin with plasma at pH 7.2 and the addition of ethanol to a concentration of 8%. Fraction 1 (FI) then precipitates. The pH of the supernatant is then reduced (horizontal line) to 6.8 and the ethanol concentration increased. This order of operations could, of course, be reversed. In regime 1, the ethanol concentration is increased to 25%, in regime 2 to 22%. The precipitate then obtained is a mixture of Cohn fractions II + III. The supernatants are then treated differently in the two regimes. In 1, the ethanol concentration is greatly increased and the pH reduced only moderately, leading to a Cohn fraction IV(4), whereafter further reduction of pH leads to fraction V and VI combined at pH 4.7 and 40% ethanol. In regime 2, the pH of the supernatant from II + III is reduced greatly and the ethanol concentration increased moderately, leading to a fraction IV(1), which is followed by precipitation of a mixture of IV(4) and V at pH 4.7 and 30% ethanol.

The regimes 1 and 2 illustrate the kind of fractionation process in which fractions II and III are taken separately from IV, and in which a fraction IV, either IV(1) or IV(4), is then taken. Particularly in processes of this kind the ranges of fractions most useful for the process of the invention comprise those of the II + III or III type lying within the area of the quadrilateral EXYZ, IV(1) type within the rectangular area AJKL and IV(4) type within the rectangular area MNCP.

In other regimes the fractionation proceeds straight from a fraction I to a fraction IV and it is therefore useful to define the pentagonal area ABCDE as one which encompasses most of the fraction IV useful in the present invention. Within this area, a IV(1) type fraction is defined by area AJKL preferred as giving slightly better results in the invention than the IV(4) type defined by area MNCP.

The Cohn fraction used must always be one which is later than fraction I, i.e. it is free of fibrins such as fibrinogen and fibronectin. Also, a fraction II per se, i.e. uncombined with III, will ordinarily be unsuitable for use in the present invention because of its high gamma-globulin content. Generally stated, the gamma-globulin content of the starting fraction should be made as low as possible. In II and III combined it is typically up to 65%. (The percentages of gamma-globulins and albumins herein are weight/weight based on total protein.) This content could be reduced by separating the fraction III material from II + III or of course by taking a fraction III out in the first place, instead of II + III. Cell growth in a medium containing a preparation based on II + III is slow and it is preferable therefore to use a fraction IV. The gamma-globulin content of a fraction IV, even when no II or III has been taken first, is unlikely to exceed 15%, and when a II or III fraction has been taken first is unlikely to exceed 5%. Such fractions are preferred for use in the present invention. Albumin is believed not to be harmful to cell growth but if excessive amounts are present in a fraction IV, the components beneficial to cell growth will become excessively diluted by the albumin. Thus, a fraction IV and V combined containing a significant amount of fraction V will contain more than 85% of albumin, which is undesirable. The fraction IV(4) + V taken in the Examples hereinafter did not give a preparation useful for supporting cell growth, presumably for these reasons. Preferably the albumin content is not more than about 75% and most preferably not more than 65%. Fraction V or fractions V and VI combined are not usable in the present invention.

The drawing refers to Cohn precipitation under ordinary ionic strengths, about 0.1 (dimensionless). Dilution of the supernatant would lower the ionic strength and therefore the concentration of ethanol required to precipitate the next fraction would also be lower. Of course, it is not normally sensible to dilute the plasma, since that only creates larger volumes to handle. However, it is to be understood that the fractions usable in the invention include their equivalents at other ionic strengths of plasma.

The fractions are produced under conditions of carefully controlled refrigeration and the precipitates are normally separated by centrifuge. They are at that time cooler than 5°C when removed from the centrifuge. The "solids" product obtained from the centrifuge is typically a very gelatinous paste, containing only about 40% dry weight solids and about 60% liquid. The liquid contains a high proportion of ethanol, typically about 20% v/v of the liquid in the case of fraction IV(1). At this stage the protein is vulnerable to being irreversibly denatured by the combined effects of high local concentrations of ethanol and low pH and is more vulnerable if the temperature is allowed to rise. It is suspected that some previous workers have not understood the severity of the environment of the proteins at this stage of processing and this might, in part at least, account for the inconsistent reports of the prior art in relation to the volume of fraction IV(4) as a component of a cell growth medium. In the present invention, the temperature is kept down, preferably to below 10°C and most preferably to about 5°C or below, until the localised concentrations of ethanol are eliminated by the ensuing homogenisation. This can be done by adding ice to the preparation.

In the homogenisation, the low temperature is initially maintained until the homogeneous state is reached. It is then safer to allow it to rise to (say) up to 25°C, although preferably the temperature is kept below 10°C until the pH is raised in the next step. The homogenisation can be carried out in any apparatus capable of effecting vigorous "creaming". If ice was added in the chilling step a few lumps will probably be visible after homogenisation.

The pH can be raised by use of any convenient strong alkali (not likely to create a toxic product), e.g. sodium hydroxide. It is necessary to raise the pH in order to dissolve the proteins. Generally stated, if the proteins are not dissolved, they will not contribute to the growth of cells. A typical pH required to dissolve the proteins is within the 6.0 to 6.5 range. As the proteins dissolve, the opaque ice-cream like material changes appearance, becoming translucent, no doubt through a re-ordering of the polymeric chains of the protein molecules into a less strained configuration. It is not necessary in this stage to raise the ph above a value effective to dissolve substantially all the protein. On the other hand, the product must in the end be brought up to a pH which is physiologically tolerable to mammalian cells. In other words, for use of the product as a cell growth medium component, the ultimate pH must be one tolerable by cells which are capable of being grown in the medium. This will depend somewhat on the kind of cell, but in general terms it is desirable ultimately to raise the pH to within the range 7.0 to 7.5, preferably about 7.2 and this is conveniently done as part of the pH-raising step for dissolving the proteins.

The remaining stages of the process are concerned with producing a sterile and physiologically acceptable product. Sterilisation cannot be carried out by heating using conventional technology and the best method applicable in the present context is filtration through a very fine filter or series of filters of progressive fineness. Before this can be done, coarse solids which would clog the filter(s) in the filtration step must be removed. Coarse solids can be separated by centrifuging. (This step is often desirable in its own right even if fine filtration is not a later processing step.) In order to centrifuge the product from step (d) effectively, it is normally sensible to dilute it first. Water can be used for this dilution, but it is more convenient to use physiological saline (aqueous sodium chloride solution buffered to impart the desired physiological pH, e.g. about 7.2, and the desired ionic strength or osmolality, e.g. about 300 milliOsmos/kg to the product). It might also be desirable to carry out a flocculation of the solids before centrifuging and this can be done by warming the preparation to a temperature of up to $30^{\circ}$C. It is then centrifuged to sediment the coarse solids and supernatant liquor is recovered from the centrifugation.

At this stage, i.e. after the pH adjustment, it might be desired to remove gamma-globulins. [They constitute about 25% by weight of total protein in a typical fraction IV(1).] Gamma-globulins might interfere with the recovery or use of proteins such as monoclonal antibodies secreted by cells into the culture medium. Typically these gamma-globulins have a molecular weight of about 145,000 (IgG). The proportion of total protein which they represent can be reduced by, for example, precipitation with polyethylene glycol, by salting out, by cold ethanol precipitation or by size exclusion chromatography. Preferably substantially all the gamma-globulins are removed. Low molecular weight material, e.g. of molecular weight below about 5,000 and preferably below about 2,000, can also be removed. Material of molecular weight of 5,000-6,000 should in any event be retained. Conveniently this can be done by concentrating the product and then dialysis against saline and filtration through a 2,000 molecular weight pore size filter. An alternative is gel permeation chromatography, using, for example Sephadex G-25 (Pharmacia Ltd.).

The preparation is then fine-filtered, ultimately going down to (say) 0.2 micrometre in size, in one or more filtration steps, as necessary. The product can be stored in sterile containers for storage at -20°C.

The product of the process is referred to herein as the "preparation of the invention". It is intended as a complete component which when added to a defined medium such as RPMI 1640, Dulbecco's modified Eagle's medium (DMEM), Ham's F-12 or a mixture of DMEM and Ham's F-12 will support growth of at least some kinds of immortal mammalian cell. In this form it will not normally support mortal mammalian cells without the addition of some other factor(s) which will enable the mortal cells to (a) adhere to a surface and (b) spread over the surface as they grow. It is generally reckoned that the substances required for those purposes include fibronectins and other proteins, e.g. fibrin and fibrinogen, collagen or polylysine. Their action can probably be improved by adding enzymes, e.g. factor XIII, particularly to promote the spreading effect. Surfaces can be pre-prepared coated with the necessary factor(s) instead of including them in the culture medium.

0143648

- 13 -

The preparation of the invention has been found to work well in conjuction with RPMI 1640 in supporting growth of a variety of immortal cells including those transformed with a virus, inherently immortal cells such as Namalwa and HEP-2 and several different hybridoma cell lines.

Accordingly, the present invention also provides a process of cultivating mammalian cells especially immortal cells, in a medium comprising a defined medium, preferably RPMI 1640, supplemented by a product of the invention. It also includes the product of such cultivation. In particular, the invention includes monoclonal antibodies obtained from hybridoma cell lines cultured in a medium containing a preparation of the invention.

The invention also provides more generally the use of a preparation of the invention in a cell growth medium for monoclonal cells. It can replace part or all of a serum component of conventional media. Normal mortal cells may require some serum component e.g. thrombin, useful as a mitogen to initiate cell division or factor XIII, as mentioned above.

While the invention has been described with reference to human blood plasma, the plasma of other animals could be used instead mutatis mutandis, particularly when the products of the cell culture are to be used for veterinary purposes.

The following Examples illustrate the invention. Temperatures are in $^{o}$C. Ratios and proportions are volume by volume unless otherwise stated.

PRODUCTION OF SOLUTIONS OF PROTEINS FROM COHN FRACTIONS OF HUMAN PLASMA PROTEINS

EXAMPLE 1

Cohn fractions were obtained from the Protein Fractionation Centre of the Scottish National Blood Transfusion Service, Edinburgh. The process of fractionation involved sequential precipitation of proteins by addition of cold ethanol to plasma at various pHs. The first fraction, I, was precipitated at pH 6.8, 8% ethanol, $-2.5^{o}$. Subsequently the next fraction, II + III, was precipitated at pH 6.7, 21% ethanol, $-5^{o}$, and the third fraction, IV(1), at pH 5.2, 21% ethanol, $-5^{o}$. In another procedure fraction II + III was precipitated at pH 6.8, 25% ethanol -5° and the third fraction, IV(4), at pH 5.85, 40% ethanol, -5°. In another variation fractions IV(4) and V combined were precipitated at pH 5.2 and 40% ethanol, -5°. The ionic strength of the supernatant liquid at each stage was about 0.1.

The protein fraction paste was removed from the centrifuge bowl in which it had been collected and transferred immediately into a tared steel bucket containing 2 kg of ice/water. The weight of the paste was calculated from the total weight of the bucket containing the paste, ice and water. Further ice/water was added so that the total weight of ice/water was the same as that of the paste. The paste was cut roughly into several pieces, the contents of the bucket quickly mixed manually and transferred to the bowl of an industrial food mixer (Hobart Ltd.). The mixture was agitated with a flat mixing paddle until there was no evidence of residual lumps of paste in the suspension, attached to the paddle or to the bottom and sides of the bowl, but before all the ice melted. The pH was taken to 7.2 by gradual addition of 1N NaOH with continuous mixing. When all the ice had thawed the preparation could be frozen at -20° and stored for further processing at a later date.

Portions of the plasma protein preparation were diluted 1:9 with water or saline (6 g/l NaCl, 0.4 g/l KC1) at room temperature, thoroughly mixed and centrifuged at about 5,000 g average R.C.F. for 30 minutes at 15°. The supernatant was collected and polished by filtration through an AP25 depth (lattice type) filter (Millipore Ltd.) and then in succession through membrane filters having pore sizes of 1.2, 0.45 and 0.22 micrometre. The final filtrate was sterilised by filtration through a sterile 0.22 micrometre filter into sterile containers. It was stored frozen at -20° until used. The total protein content (g/l) of the final product was greater than 95% of that of the preparation before centrifugation and filtration. The properties of a typical preparation (fraction IV(1), final dilution with water) were:-

| | |
|---|---|
| Total Protein | 15.2 g/l |
| Albumin | 4.5 g/l (by electrophoresis) |

- 15 -

| Transferrin | 1.2 g/l (by immunoassay) |
| Caeruloplasmin | 0.13 g/l (by immunoassay) |
| Sodium | 13 mmol/l |
| Potassium | 0.14 mmol/l |
| Citrate | 1.1 mmol/l |
| Chloride | 4.0 mmol/l |
| Phosphate | 0.4 mmol/l |
| Ethanol | 9.8 ml/l |
| pH | 7.2 |
| Conductivity | 11.39 mMho |
| Osmolality | 118 mOsmo/kg |

USE OF PRODUCTS OF THE INVENTION IN GROWING MAMMALIAN CELLS

In these Examples all the Cohn fraction preparations were prepared as in Example 1, with variations only as described.

EXAMPLE 2

A mouse - mouse hybridoma cell line ES-6 that makes antibodies to the group A antigen of human red blood cells. continues to grow and to produce antibodies in RPMI 1640 medium supplemented only with 5% v/v Cohn fraction IV(1) preparation. Cells which have been growing in this medium for 16 months have grown as well as cells maintained in medium supplemented with foetal calf serum and produce at least as much antibody (measured by haemagglutination assay). Various cell densities have been used, of between $10^3$/ml and $10^5$/ml initially, giving a final density of over $10^6$ cells/ml after about 10-15 days.

EXAMPLE 3

Epstein-Barr virus - transformed human peripheral lymphoblastoid leucocytes were prepared by the method of M. Steinitz et al., Nature 269, 420-422 (1977). These cells were raised in RPMI 1640 supplemented with 10% v/v foetal calf serum and subsequently transferred to RPMI 1640 supplemented only with 5% v/v fraction IV(1) preparation. These cells after 4 months are growing as well in the fraction IV(1) as in the foetal calf serum supplemented medium. Cell density was about $10^4$/ml initially and rose rapidly to $10^6$/ml.

## EXAMPLE 4

Two mouse hydridoma cell lines producing anti-human growth hormone immune globulins and two rat hybridoma cell lines producing anti-prolactin were grown in RPMI 1640 supplemented with 5% v/v fraction IV(1) preparation. Growth was slightly slower than in RPMI 1640 supplemented with 10% FCS, the final cell density and productivity being the same.

## EXAMPLE 5

The continuously growing human cell lines Namalwa and HEP-2 (a human tumour cell line) were transferred to medium supplemented with 5% v/v fraction IV(1) preparation and have adapted well, as judged by 3 months' growth. The cell density increased from $10^4$ to $10^6$/ml.

## EXAMPLE 6

Fraction IV(1) preparation of Example 1 was concentrated by ultrafiltration over an Amicon YM2 membrane (2,000 molecular weight pore size). The final total protein assay was 160 g/l. A series of 5 doubling dilutions (v/v) of this concentrated fraction IV(1) in physiological saline was prepared down to 1 in 32, 1 in 16 being approximately equivalent to the starting fraction IV(1) before concentration. Each of these 6 preparations was then used at 5% v/v to supplement RPMI 1640 for the cultivation of ES-6 cells. The 4 cultures thus supplemented with preparation undiluted and diluted down to 1 in 8 deteriorated fairly rapidly, the more concentrated the fraction IV(1) preparation the more rapid being the deterioration. The 1 in 16 and 1 in 32 continued to grow and to produce antibody but the 1 in 16 grew significantly more rapidly. These results indicate that the level at which the fraction IV(1) preparation has been used is close to the optimum.

## EXAMPLE 7

Fraction IV(1) prepared as in Example 1 has been further fractionated by the addition of 10% w/v of polyethylene glycol, molecular weight 4,000, (PEG-4000). This precipitates some of the material present in fraction IV(1) and in particular reduces the amount of gamma-globulin left in the supernatant. ES-6 cells grow

- 17 -

and produce antibody in RPMI 1640 supplemented with 5% v/v of the PEG-4000 treated fraction IV(1).  The growth rate is only marginally slower than when untreated fraction IV(1) of Example 1 was used.

EXAMPLE 8

Preparation from fractions II + III combined, under both sets of pH and ethanol concentration, fraction IV(4) and IV(4) and V combined were then compared in tests of growing ES-6 cells in RPMI 1640 supplemented by 5% of each of these preparations in turn.  The IV(4) showed slightly slower growth than IV(1) but similar final cell density and productivity of anti-group A antigen.  Both II and III preparations gave very much slower growth and lower final cell density but similar amounts of the antibody.  The preparation from IV(4) and V combined failed to support any cell growth or productivity at all.  The albumin content of IV(4) and V is greater than 85%, by weight of total protein.

71G

0143648

- 18 -

CLAIMS

1. A process for the preparation of a component of a cell growth medium for the cultivation of mammalian cells in vitro, which comprises:-

    (a)   extracting from mammalian blood plasma by cold ethanol precipitation a fraction of defibrinated plasma containing not more than 85% of albumin and not more than 65% of gamma-globulins both reckoned by weight of total protein in the fraction;

    (b)   chilling the extract to hold it at a temperature of below $10^{o}C$ until homogenisation by step (c) below has been carried out;

    (c)   homogenising the chilled material;

    (d)   raising the pH of the homogenate in order to re-dissolve substantially all the precipitated protein;  and

    (e)   bringing the preparation to a physiologically tolerable pH for mammalian cell growth and sterilising it.

2. A process according to Claim 1 wherein the fraction contains not more than 75% albumin.

3. A process according to Claim 2 wherein the fraction contains not more than 65% albumin.

4. A process according to Claim 1, 2 or 3 wherein the fraction contains not more than 15% gamma-globulins.

5. A process according to Claim 4 wherein the fraction contains not more than 5% gamma-globulins.

6. A process according to Claim 5 wherein the fraction contains substantially no gamma-globulins.

7. A process according to any preceding claim wherein the plasma is extracted by a Cohn type of process in which a Cohn fraction II + III is precipitated, followed by a Cohn fraction IV precipitated alone or together with Cohn fraction V.

8. A process according to Claim 7 wherein the II + III precipitate is followed by a IV(1) and then by a IV(4) together with V or is followed by a IV(4).

0143648

9.   A process according to Claim 7 or 8 wherein the plasma is extracted from a fraction defined by a matrix of pH and ethanol concentration shown in the accompanying drawing, which is a fraction II + III combined lying within the area EXYZ or a fraction IV lying within the area ABCDE.

10.  A process according to Claim 9 wherein the fraction IV lies within the area AJKL or MNCP.

11.  A process according to Claim 9 wherein the fraction IV is a fraction IV(1) in the region of point "FIV(1)" in the drawing or a fraction IV(4) in the region of the point "FIV(4)".

12.  A process according to Claim 9 wherein the plasma is extracted from a fraction III which is recovered from II+ III.

13.  A process according to any preceding claim wherein the homogenisation step (c) is carried out at a temperature of up to 5$^{o}$C.

14.  A process according to any preceding claim wherein in step (d) the pH of the homogenate is raised to a pH in the range 6.0 to 6.5.

15.  A process according to any preceding claim wherein in step (e) the preparation is brought to a pH in the range 7.0 to 7.5, before sterilisation.

16.  A process according to any preceding claim wherein in step (e) the product from step (d) is diluted with physiological saline or water before sterilisation.

17.  A process according to any preceding claim wherein sterilisation is carried out by at least one filtration through a sterile filter and the process further comprises after the pH-raising step (d) and before the filtration, separating from the liquid medium the coarse solids which would clog the filter(s) in the filtration step if not separated.

18.  A process according to Claim 17 wherein the separation of coarse solids is carried out by diluting the preparation from step (d), centrifuging the diluted prepration to sediment the coarse solids, and recovering the supernatant liquor from the centrifugation.

- 20 -

19. A process according to any preceding claim wherein gamma-globulins are removed from the preparation after step (d).

20. A process according to any preceding claim wherein material of molecular weight below about 5,000 is removed from the preparation after step (d).

21. A process of cultivating mammalian cells which comprises cultivating the cells *in vitro* in a medium comprising a defined medium supplemented with a cell growth medium component prepared by a process claimed in any one of Claims 1 to 20 and if the cells so require providing a support to which they can adhere or an adhesion-promotor in the medium.

22. A process according to Claim 21 wherein the medium is serum-free.

23. A process according to Claim 21 or 22 wherein immortal cells are cultivated.

24. A process according to Claim 23 wherein the immortal cells are hybridoma or myeloma cells.

0143648